# EUROPEAN PATENT APPLICATION

(11) **EP 1 600 777 A2**
(43) Date of publication of application: **30.11.2005**
(21) Application number: 05291129.4
(22) Date of filing: 26.05.2005
(51) Int. Cl.: G01N 33/86

(54) **A single-tube method and system for platelet function analysis**

(30) Priority: 27.05.2004 US 854708
(71) Applicant: Helena Laboratories Corporation, Beaumont Texas 77704 (US)
(72) Inventor: Ridgway, Helen Jane, Beaumont, Texas 77707 (US)
(74) Representative: Portal, Gérard

(57) **Abstract**

The present invention relates to a method for determining platelet activation. One method includes obtaining a baseline count of platelets in a sample comprising platelets in a liquid medium obtained from a physiological source of the platelets wherein the sample may include an anti-coagulant, adding a platelet activation agonist to the sample and after activation of activatable platelets in the sample, a count of unactivated platelets in the sample is then obtained and the difference between the baseline count of platelets and the count of unactivated platelets is determined. This difference is a measure of the activity of the platelets in the original sample. The present invention further relates to a kit for determining platelet activation.

## Description

### BACKGROUND

### Technical Field

A method and a test kit for determining platelet function such as a determination of platelet function (aggregation, agglutination, and adhesion). Platelet function may be determined using a differential cell counting technique.

### Background of the Technology

Platelets are biological cells in blood circulation that provide the first line of hemostatic defense. They contribute the initial physiology and biochemistry to maintain normal circulatory integrity to help prevent exsanguination or bleeding to death upon injury, especially venous or vascular injury. Life cannot be maintained without adequate platelet numbers and without some meaningful degree of platelet functionality or quality.

Platelets are irregularly-shaped, colorless bodies which are normally present in blood at the level of about 250,000 cells per mm. Their sticky surfaces, along with other endogenous substances or stored chemicals, act to form primary platelet plugs and ultimately, stable blood clots to stop or arrest bleeding. When bleeding from a wound suddenly occurs, the platelets stick to the wound site and release substances that cause them to gather (aggregate), en masse at the venous injury. This prevents excessive blood from escaping the vasculature, thus preventing irreversible morbidity and mortality. Other coagulation proteins in the blood in concert with the platelets form a fibrin clot usually within minutes.

Coagulation (or blood clotting) and platelet activation, adhesion, and aggregation occur when blood is exposed to non-biological material including any material that is dissimilar to venous endothelium and more importantly, biological materials such as an injured blood vessel. Often, during the routine practice of medicine, blood may be exposed to a hostile, platelet-activating environment such as contact with an extracorporeal blood circuit or the results of invasive procedures that injure the vascular lining or exposure of the blood to air. The platelets normally respond to this condition and begin to activate. After activating, the platelets react with specific coagulation plasma proteins and fibrinogen to begin forming fibrin, tiny thread-like visible strands of protein. These fibrin threads link to form a web-like mesh that traps red cells, white cells, and platelets, leading to the formation of a stable or insoluble clot. On the skin surface, the blood clot is ultimately transformed from an initial plug to arrest bleeding to a healing process in which the blood clot becomes a crusty protective layer of cells (scab).

Platelets that have the ability to activate are commonly called "functional" platelets. The extent to which these platelets activate or perform qualitatively is variously called platelet activity, platelet function, platelet aggregation, or platelet adhesion. Once the platelet has performed its qualitative aggregation function fully, the endogenous biochemistry has been consumed and cannot be recharged or revitalized. The sticky or adhesive quality of the platelet may still provide hemostatic support in microcirculatory physiology, but the biochemical aggregating quality of the platelet is a one time event or occurrence. Platelet quality is also affected both positively and negatively by contemporary over-the-counter drugs and by hospital-based pharmacology compounds. Platelet function characteristics may also be manipulated by certain agents to better control specific medical procedures and surgeries. Some compounds are used to slightly alter platelet function by causing intentional temporary dysfunctionality, as in the case of aspirin therapy, for heart disease patients who are more prone to thrombosis or clot. Aspirin is used to minimize platelet adhesion and cause qualitative platelet defects that are nevertheless beneficial to patient well-being.

Clinically, platelet assessment is a very useful parameter and provides relevant information regarding a patient's hemostatic or bleeding status and thrombotic state.

Even though platelets are uniquely associated with, and are a contributor to thrombosis, a leading cause of morbidity and mortality, the technology to measure and predict platelet physiology is lacking and sorely needed. There are a very limited number of ways (mostly unsatisfactory) to measure platelet function both qualitatively and quantitatively. Notable accepted laboratory methods include bleeding time and platelet aggregometry. Each of these techniques are discussed below.

Bleeding time is a qualitative and not a quantitative measure. In this procedure, a small invasive incision is made in the forearm after placing a blood pressure cuff on the same arm and inflating it to 40 mm Hg. As blood exudes from the wound it is blotted with filter paper, and the time at which bleeding stops is recorded. The normal bleeding time is usually less than 9-10 minutes. The bleeding time test is commonly performed as part of the pre-operative patient screen. The test is laborious and expensive and the time and personnel requirements prevent this test from being performed routinely or even effectively in the operating room.

Platelet aggregometry is a quantitative platelet measurement test and is generally considered to be the reference method. This test or assay measures the level or percent of functionality platelet activity in patient plasma and is reported in percent platelet aggregation. The assay is performed by briefly pre-incubating normal human platelet-rich plasma and adding a known platelet aggregation agent (*e.g.*, ristocetin, collagen, etc.) in a traditional platelet aggregometer. Aggregometry works on a simple photometric principle and does not use a numeric counting technique. The amount of light that passes through a platelet-rich plasma sample in aggregometry is low and is electronically calibrated to zero. This is compared to maximum or 100 percent light transmission through platelet-poor plasma (sometimes called platelet-free plasma) due to the lack of light absorption by the platelets.

By adding a platelet aggregating agent to the platelet-rich plasma, the platelets are caused to clump or aggregate and separate from the liquid phase. Light transmission thus increases as the platelet-rich plasma sample becomes more translucent as compared to the 100 percent light transmission of control platelet-poor plasma. The principle of this test is that the interaction between the aggregating agent and the platelets causes the activation of the platelets, subsequently leading to platelet activation, adhesion, and aggregation, or simple "clumping". The functional platelets are thus trapped in the platelet aggregate or "clump". This clumping allows an increasing proportionate level of light to now pass through the platelet-rich plasma patient sample. The difference in the two samples (pre-clump and post-clump) are compared as a percentage. The level of functional platelets percent aggregation is determined by comparison of the percent difference between light transmission of platelet-poor plasma and that of platelet-rich plasma following the addition of the known aggregating agent.

Normal platelets and disease or damaged platelets are accurately characterized by using a variety or combination of chemicals or known aggregating agents. When these agents are used in known concentrations, an accurate depiction of disease and seriousness of platelet damage or dysfunction can be identified when using the aggregometer. There are numerous platelet adhesion and aggregating agents with differing platelet response. Concentrations of aggregating agents has become specific to diagnostic, disease, and dysfunctionality.

The adhesion or "sticky" quality of platelets can also be measured using glass beads as a reagent because platelets have an affinity to glass and platelet adhesion has a linear response to glass and glass-like materials (*e.g.*, fiberglass). In general, this test involves running platelets over a glass bead column, collecting the run-through and determining the number of platelets that adhere to the glass bead surfaces as a percentage of the total platelets allowed to flow over the beads. This technique is not commercially available and as such has not reached any satisfactory level of acceptance by the art. This characteristic is nevertheless uniquely important following coronary bypass surgery as micro vascular bleeding is common and the adhesion quality of platelets is vital when arresting tiny vessel or capillary and capillary-like bleeding.

These platelet aggregometry and adhesion procedures are arduous, time-consuming, expensive, and require tedious blood specimen collection, handling, and processing procedures. Consequently, these techniques are error prone. Another complicating aspect is the unstable nature of platelet adhesion and function (or aggregation). The aggregation test is largely performed in only the more advanced or specialty hemostasis laboratory environments and as such, platelet function testing is rarely performed even though platelet viability is a major and routine indication for blood transfusion, including emergency transfusions.

Consequently, most blood and blood platelet transfusions are given without a platelet functionality indication or laboratory support. Platelet aggregometry is typically reserved for the diagnosis of a rare congenital bleeding disorder and is not often used to better transfuse blood and blood platelets regardless of the indications and recommendations for platelet transfusion. Further, platelet aggregometry is not typically offered as a STAT test and is most often a scheduled test by appointment with a laboratory.

Prior to modem electronics, hematological blood cell counting (commonly called the CBC or complete blood count) was done manually and with relative accuracy regarding red and white blood cells. The reference method for red cells was the spun haematocrit and for white cells it was a staining technique that was then read on a phase microscope. Platelet counts were less satisfactory using manual methods or microscopes because of their small size and instability (activating or clumping). Electronic cell counting enjoyed early success regarding red cells and white cells, but platelet counts were more problematic for the same reasons as mentioned above regarding manual counts. Contemporary hematology analyzers or cell counters are sophisticated and highly reliable high capacity multichannel devices. They typically employ the technique of measuring changes in electrical impedance as the cells and platelets flow through a small aperture with computer analysis of the electrical signals generated. In effect, the cell counter identifies a cell type *(i.e.,* a white cell, a red cell, or a platelet) by size, shape, and mass. A red cell has a diameter of approximately 7.5 microns, and platelets are elongated measuring approximately 3 microns in length and 1 micron in thickness. White cells are larger than red cells and will range in sizes typically above 7 microns to over 20 microns and will vary in shape from multi-lobed to spherical, and non-uniform to almost round. Commonly called the Coulter Counter (also a branded product) or Coulter Principal, the electronic cell counter technologies are manufactured by numerous companies today including Coulter Electronics, Miami, Florida (U.S.A.), Abbott Laboratories, Chicago, Illinois (U.S.A.), ABX, France, and others. Routine CBC analysis is a widely-performed multi-parameter biologic test.

The CBC instruments and methodology described above are referred to as electrical impedance cell counters (EICC) or simply CBC instruments, by which terms are meant the art-known passage of cellular blood components, in a dilute medium through an aperture and the numeric counting of cells by reason of the changes caused in the electrical conductivity of the medium as the cells pass the measuring electrodes. While electrical impedance cell counters are very effective to count platelets, no information is obtained on platelet function, that is, the ability of the platelets to exert their required function in body physiology. Remarkably, platelet functionality and "stickiness" characteristics have always been considered a problem regarding cell counting, and the preservatives used to collect whole blood for blood cell counting are designed specifically to disable the functionality and adhesion characteristics of platelets. This blood collection preservative is EDTA (ethylene diamine tetracetic acid) and is used worldwide in standard blood collection test tubes (often referred to as simply a purple-top blood collection tube). Blood and blood platelets preserved in EDTA will not respond to aggregating or adhesion agents or medium.

As noted above, one is able to count platelets on EDTA-preserved blood using the CBC counter. One cannot determine platelet function, however, using the counting technique in the CBC counter in the presence of EDTA because the EDTA prevents the platelets from aggregating even in the presence of activating agents or agonists. Those skilled in the art are well aware that EDTA prevents aggregation of the platelets in the presence of agonists. Therefore, it has been thought that while the CBC instrument is eminently useful in counting cells, it is not useful in determining platelet function.

In the CBC determination, the starting material is diluted whole blood, generally preserved with EDTA. Those skilled in the art have recognized that EDTA alters the platelet function in such a way as to preclude measurement via activation by platelet agonists. Therefore, it is not possible to induce activation of platelets to cause clumping in the presence of EDTA. The art has not heretofore found a way to determine the activity of platelets in whole blood and then to evaluate the number of platelets which can be activated largely because the CBC instrument process has been restricted to the use of EDTA.

### SUMMARY OF THE INVENTION

In a non-limiting overview, a method for measuring platelet function is provided which comprises one or more of: obtaining a baseline count of platelets in a sample comprising platelets in a liquid medium obtained from a physiological source of the platelets, wherein the sample is devoid of an agent which produces exogenous platelet activation; adding an activation agonist to the sample; allowing activatable platelets in the sample to activate; obtaining a count of the unactivated platelets in the sample after activation of the activatable platelets; and determining the difference between the baseline count of platelets in the sample and the count of unactivated platelets in the sample. The difference is a measure of the activity of the platelets in the original sample.

In all the methods, prior to addition of the agonist, the sample may include a preservative or anti-coagulant which does not produce exogenous platelet activation or, if any such substance is present, it is not present in an amount which is effective to produce such exogenous platelet activation. In a non-limiting example, the sample may include D-phenylalanyl-L-prolyl-L-arginine chloromethyl ketone (PPACK) as an anti-coagulant prior to addition of the agonist. The count of platelets can be obtained in an electrical impedance cell counter when a differential cell count or the like is desired. In a non-limiting example the platelet activation agent can be adenosine 5' di-phosphate (ADP), collagen, ristocetin, epinephrine, arachidonic acid, or thrombin receptor activating peptide (TRAP). The platelets may be human platelets.

A kit for use in obtaining platelet counts is provided. The kit comprises a preservative or anti-coagulant which does not produce exogenous platelet activation (or, if any such substance is present, it is not present in an amount which is effective to produce such exogenous platelet activation) in a first container and a platelet activation agonist which is external to the interior of the first container, e.g., in a second container. The preservative or anti-coagulant which does not produce exogenous platelet activation may be D-phenylalanyl-L-prolyl-L-arginine chloromethyl ketone (PPACK) or sodium citrate. Exemplary platelet activation agonists include, but are not limited to, adenosine 5' di-phosphate (ADP), collagen, ristocetin, arachidonic acid, or thrombin receptor activating peptide (TRAP).

Various combinations of preservatives and agonists may be used except those combinations which may be specifically excluded in the following description..

Within the context of the present invention, the meaning of the terms "platelet activation agent", "platelet activation agonist", "agonist", or "activation agent", as used throughout the present text, is the same, as is understandable to the person skilled in the art.

### DETAILED DESCRIPTION OF THE INVENTION

A method in which a counting technique is used to determine platelet function is described. The method is versatile enough to permit platelet function analysis to be performed on a CBC instrument without changes in hardware and by using a simple arithmetic equation which is described more fully below.

A method for determining platelet function is provided which involves the use of a first container, such as a sample tube into which is placed a suitable blood sample. The tube comprises the blood sample and, optionally, an anticoagulant or preservative which does not interfere with platelet function. If there is a concern that the blood may tend to coagulate before a baseline count can be taken, the a preservative should be added. Preservatives such as EDTA, which are known to interfere with platelet function, are not present in the sample or, if present, are present in an amount which is sufficiently small so as to avoid affecting platelet function.

The preservatives or anti-coagulants may initially be provided in the first tube or container, or in a different container and may be added prior to or after the blood sample is introduced into the tube. If a preservative is to be used, it is of course more convenient to place such preservative in the tube prior to the introduction of the blood sample.

By way of illustration only, sodium citrate and D-phenylalanyl-L-prolyl-L-arginine chloromethyl ketone (PPACK) are blood preservatives which do not interfere with induction or initiation of platelet activation by platelet agonists. If PPACK is utilized, it may be present at a concentration of 50 - 75 micromolar, but the foregoing concentration range is merely exemplary.

If a platelet count is needed, the platelets in the sample are then counted (*e.g.*, on a CBC instrument) to obtain a baseline count.

A subsequent step is adding a platelet activation agonist to the sample in the first container to produce exogenous platelet activation. The agonist may be provided in another container or at least is initially maintained exterior to (outside of) the interior of the first container.

Again for illustrative purposes, exemplary platelet activation agents include adenosine 5' di-phosphate (ADP), collagen, ristocetin, arachidonic acid, epinephrine, adrenaline, and thrombin receptor activating peptide (TRAP). When PPACK is the preservative, ADP functions well as the agonist or activation agent.

After the activation agonist is added to the sample, the activatable platelets in the sample are allowed to aggregate. If a P.T. or P.T.T. test is being performed, the interval during which the aggregation or coagulation occurs is timed. Within the context of the present invention, the term "P.T." refers to Prothrombin Testing" : this is a test for the amount of time it takes for blood to clot when a test is made for blood Factors II, VII, VIII, and X.

Further, within the context of the present invention, the term "P.T.T." refers to "Partial Prothrombin test time" : this is another test for determining the length of time it takes for blood to clot testing specifically for clotting Factors VII, VIII, XI, XII.

If the platelets are to be counted, then (and if a CBC hematology instrument for platelet counting is being used) the tube containing the platelets can be placed into a CBC hematology instrument for counting the platelets.

It is known when a platelet count is desired that once the whole blood or diluted whole blood and agonist are present in the tube, the contents of the tube are vigorously mixed end-to-end usually for about 30 seconds to several minutes depending on the constituents to allow the agonist to induce the activity of the platelets and initiate the clumping of the active platelets. Naturally, it is preferred that the amount of agonist and the length of time employed in activation be sufficient to activate the maximum number of platelets in order to achieve accurate measurements. The temperature is normally from room temperature to normal body temperature as required by the testing environment. It should be noted, of course, in view of what has been said previously about the function of EDTA and its deleterious effect on the activity of the platelets in the presence of an agonist, that the tube should not contain any detrimental amounts of EDTA or any similar materials having a depressant effect on platelet activation.

Platelet adhesion may be measured using the same technique and by having a glass bead agonist or another, glass-like particle or other material platelet attracting substance that causes the platelet to "stick" or adhere to the material in a controlled, predictable manner. The platelets that are counted after aggregation are those which do not stick to or aggregate to any other platelets and are therefore seen and counted by the cell counter. Platelets counted after activation are therefore non-sticky and non-functional.

The platelet counter uses the difference in the numeric count of platelets obtained from an initial inactivated sample of human or animal platelets from the count of platelets left in the sample after an agonist for activating the function of the platelets has been added to cause the platelets to clump or aggregate or adhere. In a cell counter, two platelets that have aggregated, joined or clumped together will generally measure over 6 microns and may be mis-characterized as either a red cell or a white cell, but seldom a platelet. Since the platelet is the smallest cell that can be counted in a CBC instrument, two or more platelets joined or clumped together will usually be measured as something other than a platelet. From a platelet functionality perspective, the fact that two or more platelets aggregated or clumped together becomes a specific and direct measure of platelet viability, aggregation, and/or adhesion. Further, the cell counter may be programmed to look for only unaggregated platelets and to ignore all other particles or cells larger than platelets. In this manner, the counting procedure disregards the clumped aggregates and will only report, assuming it is properly so programmed, the unclumped platelets. The clumped platelets thus represent the active platelets and the percent of functional platelets is easily determined from the counting procedure by dividing the difference between the baseline and aggregated count by the baseline count.

While the foregoing describes a CBC platelet counting technique, it should be emphasized that platelet function may be obtained using any platelet numeric counting methodology as long as the platelets count in the sample after activation is compared to any appropriate baseline numeric count within a suitable time frame irrespective of the method of counting.

As a further exemplary, non-limiting description of the agonists, ADP can be added to the sample at a final concentration of 5 to 20 micromolar. When collagen is used, the collagen can be added to the sample at final concentration of 1 to 10 microgram/ml. When ristocetin is employed as an agonist, the ristocetin can be used, for example, at a concentration of 0.50 - 1.5 mg/ml. The above concentrations are merely exemplary, however, and other concentrations of these agonists can also be used.

The agonist can be provided in solid form. For example, ADP can be provided in dry or solid form (*e.g.*, in a dropper bottle). The dry ADP can be added directly to the sample. Alternatively, the dry ADP can be reconstituted, preferably with water, before use. The agonist can also be provided in liquid form. Collagen, for example, can be supplied as an agonist in liquid form (*e.g.*, in a liquid dropper bottle).

To the extent it is helpful for an understanding of the methodology, it should be explained that the normal CBC instrument platelet counting procedures performed on the sample results in a delta between the platelet baseline count and the count obtained after platelet activation. The delta is used to determine a number characterizing the number of functional platelets in the sample. Activated platelets are not available for the platelet count and, therefore, a simple delta between the counts can give the level of platelet functionality in a sample. For example, if the baseline count is 276,588/mm³ and an unactivated platelet count of 32,000/mm³ measured by cell counter after activation and platelet aggregation, the result is [(276,588 - 32,000) divided by 276,588] x 100 = 88 %, which is 88% platelet aggregation.

The technique as described may be expanded and may be employed to diagnose various platelet dysfunctions. For example, once a platelet activity is determined on a sample, various reagents can be added to additional tubes in order to assess the effect of different agents on the activation of platelets. Accordingly, it is possible to evaluate the efficacy of antifibrinolytic or platelet protectorate such as aprotinin and transexamic acid, DDAVP, aminocaproic acid, (epsilon-aminocaproic acid) and aspirin to determine the levels, if any, of inhibition, suppression or enhancement these products have on the ability of the platelets to function when stimulated. In a similar manner, ADP Inhibitors and IIb-IIIa anti-platelet compounds can be measured therapeutically when using this approach. Likewise, patients who have congenital or acquired platelet disorders can be diagnosed and
characterized. The technique as described above can thus be used to determine accurate dosages of the *in vivo* use of some of the above-mentioned compounds. The technique also contemplates having additional tubes or containers which include one or more of such materials to be supplied for use on the hematology cell counting instruments. As an example, to diagnose storage pool disease (a well-articulated platelet disorder), a combination of aggregating agents like collagen, epinephrine, ADP, and ristocetin in specific concentrations would provide differing aggregation responses such that a differential diagnosis may be offered. Similar yet more dramatic circumstances would be the differential diagnosis of bleeding post-coronary bypass surgery, which could result in precise transfusion information such that the correct blood component could be prepared and infused.

The present technique has been generally described in its method or process modality and will now be further explained in the product or test kit modality.

As noted above, a first test tube or container may be provided for the sample. A preservative which does not interfere with platelet function (or one which may interfere with platelet function to a known degree and therefore can be factored out of any results obtained) may be initially included in the first container or may be provided external to the interior of the test tube, e.g., in a second container. The agonist may be provided in a third container or at least external to the interior of the first container. Container, as used herein, is not restricted to a test tube, nor to any physical shape, nor to any material. For example, if the agonist is to be provided in dry form, it may be stored in a paper packet. The agonist and anti-coagulant can be supplied in lyophilized form or may be in the form of physiologic saline solutions or suspensions thereof. The platelet activation agonist can be provided in liquid or solid form.

The actual amounts of materials, concentrations, dilutions, and the like are well-known in the hemostasis and cell counting field and are easily determined and adjusted depending upon the user's particular preferences and the objectives sought. For example, a typical CBC instrument cell counter dilutes a whole blood sample by adding 1 part thereof to 183 parts of physiologic saline. Then 27.5 µl of this dilution are mixed with 3 ml of physiologic saline as the diluent resulting in a dilution of 1 /20,000. This is the sample upon which the counts are obtained.

Exemplary reagents, concentrations, and volumes are the following:

### I. Agonists:

| | | |
|---|---|---|
| 1. | Collagen, aqueous solution, 2 mg/ml | diluted 1 part with 19 parts of saline (100 µg/ml) to make a collagen stock solution |
| 2. | ADP (2x10⁻⁴ mol/L) | |
| 3. | Epinephrine (1x10⁻⁴ mol/L) | dilute 1 part to 9 parts of saline to make a stock solution in water |
| 4. | Ristocetin (15 mg/ml) | |

### II. Whole Blood Dilution (prior to testing)

whole blood diluted 1:1 with saline

### III. Volumes for testing:

| | | |
|---|---|---|
| 1. | Collagen: | 1 ml of blood dilution from II + 500 µl of Collagen Stock solution (100 µg/ml) |
| 2. | ADP: | 3 ml of blood dilution from II + 25 µl ADP |
| 3. | Epinephrine: | 2 ml of blood dilution from II + 20 µl |
| | | epinephrine stock solution |
| 4. | Ristocetin: | 1 ml of blood dilution from II + 60 µl ristocetin solution |

The above are not to be considered as limiting the amounts of reagent or the reagent concentrations and volumes, but are only illustrative. These amounts may vary within wide ranges depending upon the particular modes of testing involved and the objectives sought in the testing. Those skilled in the art are capable of selecting such variables in consideration of optimization of the procedures.

While the foregoing specification teaches the principles, with non-limiting examples provided for the purpose of illustration, it will be appreciated by one skilled in the art from reading this disclosure that various changes in form and detail can be made.

## Claims

1. A method for measuring platelet function comprising:
obtaining a baseline count of platelets in a sample comprising platelets in a liquid medium obtained from a physiological source of the platelets, wherein the sample is devoid of an effective amount of an agent which interferes with platelet function;
adding a platelet activation agonist to the sample;
obtaining a count of the unactivated platelets in the sample after activation of the activatable platelets;
determining the difference between the baseline count of platelets in the sample and the count of unactivated platelets in the sample;
wherein the difference is a measure of the activity of the platelets in the original sample.

2. The method of Claim 1, wherein the sample prior to the addition of the platelet activation agonist is devoid of EDTA.

3. The method of Claim 1, wherein the sample prior to the addition of the platelet activation agonist includes an anti-coagulant which does not interfere with platelet function.

4. The method of Claim 3, wherein the sample prior to the addition of the platelet activation agonist includes D-phenylalanyl-L-prolyl-L-arginine chloromethyl ketone (PPACK) as an anti-coagulant.

5. The method of Claim 3, wherein the sample prior to the addition of the platelet activation agonist includes sodium citrate as an anti-coagulant.

6. The method of any one of claims 1 to 5, wherein the platelet activation agonist is in solid form and wherein the platelet activation agonist is added in solid form to the sample.

7. The method of any one of claims 1 to 5, wherein the platelet activation agonist is in solid form, the method further comprising reconstituting the platelet activation agonist, preferably with water, prior to adding the platelet activation agonist to the sample.

8. The method according to any one of claims 1 to 7, wherein the count of platelets is obtained according to a platelet numeric counting methodology wherein the platelets count in the sample after activation is compared to an appropriate baseline numeric count within a time frame which is sufficient to allow activatable platelets in the sample to be activated.

9. The method of Claim 8, wherein the count of platelets is obtained in an electrical impedance cell counter.

10. The method of any one of claims 1 to 9, wherein the sample is devoid of any agent which interferes with platelet function.

11. The method of any one of claims 1 to 10, wherein the platelet activation agonist is selected from the group consisting of adenosine 5' di-phosphate (ADP), collagen, ristocetin, epinephrine, arachidonic acid, and thrombin receptor activating peptide (TRAP).

12. The method of Claim 11, wherein the agonist is adenosine 5' di-phosphate (ADP).

13. The method of Claim 12, wherein the ADP is present in the sample at a concentration of 1 to 25 µM.

14. The method of Claim 13, wherein the ADP is present in the sample at a concentration of 5 to 20 µM.

15. The method of Claim 11, wherein the agonist is collagen.

16. The method of Claim 15, wherein the collagen is present in the sample at a concentration of 1 to 50 µg/ml, preferably 1 to 35µg/ml.

17. The method of claim 16, wherein said concentration of collagen is about 33 µg/ml.

18. The method of Claim 16, wherein the collagen is present in the sample at a concentration of about 2 µg/ml.

19. The method of Claim 16, wherein the collagen is present in the sample at a concentration of about 10 µg/ml.

20. The method of Claim 11, wherein the agonist is ristocetin.

21. The method of Claim 20, wherein the ristocetin is present in the sample at a concentration of 0.75 to 1.0 mg/ml.

22. The method of any one of claims 1 to 21, wherein the platelets are human platelets.

23. The method of any one of claims 1 to 22, wherein the sample comprises a blood preservative which does not interfere with platelet function.

24. A test kit comprising:
a first container to receive a sample;
an anti-coagulant which does not interfere with platelet function; and
a platelet activation agonist external to said first container.

25. The kit of Claim 24, wherein the anti-coagulant which does not interfere with platelet function is D-phenylalanyl-L-prolyl-L-arginine chloromethyl ketone (PPACK).

26. The kit of Claim 24, wherein the anti-coagulant which does not interfere with platelet function is sodium citrate.

27. The kit of any one of claims 24 to 26, wherein the platelet activation agonist is selected from the group consisting of adenosine 5' di-phosphate (ADP), collagen, ristocetin, arachidonic acid, and thrombin receptor activating peptide (TRAP).

28. The kit of any one of claims 24 to 27, wherein the platelet activation agonist is in liquid form.

29. The kit of any one of claims 24 to 27, wherein the platelet activation agonist is in solid form.

30. The kit of Claim 24, wherein the platelet activation agonist is adenosine 5' di-phosphate (ADP).
